# EUROPEAN PATENT APPLICATION

(11) **EP 3 473 641 A1**
(43) Date of publication of application: **24.04.2019**
(21) Application number: 17813425.0
(22) Date of filing: 16.06.2017
(51) Int. Cl.: C07K 7/06, A61K 39/00, A61P 31/04, A61P 31/12, A61P 35/00, A61P 37/04, C07K 7/08, C12N 15/09

(54) **NUCLEIC ACID-ANTIGEN PEPTIDE CONJUGATE**

(30) Priority: 16.06.2016 JP 2016120261
(71) Applicant: NapaJen Pharma, Inc., Burlingame, California 94010 (US)
(72) Inventor: MOCHIZUKI, Shinichi, Koganei-shi Tokyo 184-8588 (JP); SAKURAI, Kazuo, Koganei-shi Tokyo 184-8588 (JP); HIGUCHI, Sadaharu, Koganei-shi Tokyo 184-8588 (JP); ANDO, Hironori, Koganei-shi Tokyo 184-8588 (JP)
(74) Representative: Müller-Boré & Partner Patentanwälte PartG mbB
(86) International application number: PCT/JP2017/022283
(87) International publication number: WO 2017/217531

(57) **Abstract**

The purpose of the present invention is to provide a nucleic acid-antigen peptide conjugate that is capable of forming a complex with polysaccharides that have a β-1,3-glucan skeleton such as schizophyllan, and can efficiently present the antigen peptide in an antigen-presenting cell. A conjugate bonded, via a linker added to polydeoxyadenine and a disulfide bond, to an N-terminal cysteine residue of an antigen peptide that has eight or more amino acid residues having a cysteine residue at the N-terminal is capable of forming a complex with polysaccharides that have a β-1,3-glucan skeleton, and can efficiently present the antigen peptide in an antigen-presenting cell.

## Description

### TECHNICAL FIELD

The present invention relates to a nucleic acid-antigen peptide conjugate. More specifically, the present invention relates to a nucleic acid-antigen peptide conjugate which is capable of forming a complex with a polysaccharide having a β-1,3-glucan skeleton such as schizophyllan, and which can efficiently present the antigen peptide in an antigen-presenting cell.

### BACKGROUND ART

The basic principle of prevention of infectious diseases and cancer by vaccines is based on artificial pseudo-infection to induce acquired immunity, and to induce cellular immunity and antibody production against a specific pathogen. In cell-mediated immunity, first, an antigen-presenting cell phagocytoses an antigen protein and digests the antigen protein with an intracellular protease to generate an antigen peptide. The antigen peptide binds to MHC, and the antigen peptide is exposed on the membrane surface of the antigen-presenting cell. Then, information of the foreign substance is presented to T cells. Subsequently, the T cells are activated by recognizing the antigen peptide bound to MHC by the T cell receptor on the membrane surface. The activated T cells specifically damage cancer cells or virus-infected cells or induce the production of various cytokines, thereby activating immunity.

There are two types of MHC molecules, class I and class II, which bind peptides at different locations in the cells. Peptides generated in the cytoplasm migrate to the endoplasmic reticulum, bind to MHC class I molecules, and migrate to the cell surface. Then, the peptides presented as antigen peptides are recognized by CD8T cells. Further, proteins taken up by endocytosis are digested with lysosome, the resulting proteins are bound to MHC type II molecules in the endoplasmic reticulum and migrated to the cell surface. Then, the proteins presented as antigen peptides are recognized by CD4T cells.

On the other hand, there is a conventionally known method of forming a triple helix complex composed of one molecule of nucleic acid and two molecules of β-1,3 glucan in which β-1,3 glucan (e.g., schizophyllan) which forms a triple helix structure in nature is dissolved in an aprotic nonpolar organic solvent or an alkaline solution of 0.1 N or more to cause disassociation of the triple helix into a single strand, a single-stranded nucleic acid is added to the resulting mixture, and the solvent is replaced with water or the pH of the alkaline solution is adjusted to neutral. By complexation of the nucleic acid and β-1,3 glucan in the above manner, the nucleic acid may be delivered into the cell while suppressing hydrolysis of the nucleic acid molecule by nucleases, and while suppressing undesirable interactions between the nucleic acid molecule and proteins in the body such as non-specific binding of nucleic acid molecules with proteins in the blood plasma.

Therefore, various attempts have been made to utilize β-1,3 glucan in the vaccine therapy in which antigen peptides are administered, from the viewpoints of improving the delivery efficiency of antigen peptides to antigen-presenting cells and improving immunostimulatory activity. For example, Non-Patent Document 1 discloses that, in the case of using a ternary complex of β-1,3 glucan/antigen peptide/CpG DNA obtained by bonding β-1,3 glucan to an antigen peptide and further complexing CpG DNA with the β-1,3 glucan, the antigen peptide and CpG DNA functioning as an adjuvant can be simultaneously and efficiently delivered to the antigen-presenting cell. Further, Patent Document 1 discloses that a peptide/polynucleotide conjugate in which an antigen peptide is bonded, via a covalent bond, to a polynucleotide or a polynucleotide derivative is complexed with β-1,3 glucan to form a complex, whereby the productivity is excellent and the complex has high immunostimulatory activity.

### PRIOR ART DOCUMENTS

### NON-PATENT DOCUMENT

Non-Patent Document 1: Synthsis and in Vitro Characterization of Antigen-Conjugated Polysaccharide as CpG DNA Carrier, N. Shimada, K. J. Ishii, Y. Takeda, C. Coban, Y. Torii, S. Shinkai, A. Akira and K. Sakurai, Bioconjugate Chem., 17, 1136-1140 (2006)

### PATENT DOCUMENT

Patent Document 1: International Publication No. WO 2015/118789

### SUMMARY OF THE INVENTION

### PROBLEMS TO BE SOLVED BY THE INVENTION

It has been clarified that Dectin-1 is expressed in antigen-presenting cells such as macrophages and dendritic cells, and further that a nucleic acid-polysaccharide complex containing β-1,3 glucan (such as schizophyllan) has the property to be specifically incorporated into Dectin-1 expressing cells. Therefore, as in Non-Patent Document 1 and Patent Document 1, a complex containing β-1,3 glucan and antigen peptide has excellent delivery efficiency to antigen-presenting cells, and is greatly expected to be used in the vaccine therapy.

On the other hand, when a heterologous molecule such as polysaccharide or nucleic acid is bonded to an antigen peptide, the antigen peptide cannot be bonded to MHC after being incorporated into the antigen-presenting cell, whereby the antigen peptide is not presented on the membrane surface of the antigen-presenting cell or the efficiency of presenting the antigen peptide may be reduced. In Non-Patent Document 1 and Patent Document 1, there has been no adequate study as to whether the antigen peptide can be efficiently presented in the antigen-presenting cell when the antigen peptide is bonded to the heterologous molecule in any bonding mode. Indeed, the present inventors have confirmed that the technique of Patent Document 1 requires a relatively large number of peptides to induce the response of cytotoxic T cells (CTL) and cannot efficiently induce antigen-presenting cells (see Examples 5 and 6 to be described later).

An object of the present invention is to provide a nucleic acid-antigen peptide conjugate which is capable of forming a complex with a polysaccharide having a β-1,3-glucan skeleton such as schizophyllan, and which can efficiently present the antigen peptide in an antigen-presenting cell.

### MEANS FOR SOLVING THE PROBLEM

The present inventors have conducted intensive studies to solve the above-mentioned problems. As a result, they have found that a conjugate, where an N-terminal cysteine residue of an antigen peptide that has a cysteine residue at the N-terminal and has eight or more amino acid residues is disulfide-bonded to a linker added to polydeoxyadenine, can efficiently present the antigen peptide in an antigen-presenting cell. In addition, such a conjugate is capable of forming a complex with a polysaccharide having a β-1,3-glucan skeleton such as schizophyllan, since polydeoxyadenine exists as a single strand. The present invention has been completed by further repeated studies based on such knowledge.

That is, the present invention provides the following aspects:
1. A nucleic acid-peptide conjugate, where an N-terminal cysteine residue of an antigen peptide that has a cysteine residue at the N-terminal and has eight or more amino acid residues, is disulfide-bonded to a linker added to polydeoxyadenine.
2. The nucleic acid-peptide conjugate according to 1, where a C-terminal amino acid residue of the antigen peptide is a hydrophobic amino acid residue.
3. The nucleic acid-peptide conjugate according to 1 or 2, where the antigen peptide is a peptide presented by an MHC class I molecule.
4. The nucleic acid-peptide conjugate according to any one of 1 to 3, where at least a part of a phosphodiester bond of the polydeoxyadenine is phosphorothioated (S-modified).
5. A complex comprising: the nucleic acid-peptide conjugate according to any one of 1 to 4; and a polysaccharide having a β-1,3-glucan skeleton.
6. A vaccine formulation comprising: the nucleic acid-peptide conjugate according to any one of 1 to 4; or the complex according to 5.
7. An immunization method comprising administering the nucleic acid-peptide conjugate according to any one of 1 to 4 or the complex according to 5, to a subject who needs to be given immunity or needs to enhance immunity.
8. Use of the nucleic acid-peptide conjugate according to any one of 1 to 4, or the complex according to 5, for the vaccine formulation.

### ADVANTAGES OF THE INVENTION

A N-terminal cysteine residue of an antigen peptide that has a cysteine residue at the N-terminal and has eight or more amino acid residues is disulfide-bonded to polydeoxyadenine via a linker, whereby the conjugate can efficiently present the antigen peptide in the antigen-presenting cell.

Further, in the conjugate of the present invention, a polysaccharide having a β-1,3-glucan skeleton such as schizophyllan can be complexed with a moiety of polydeoxyadenine. By complexation of the conjugate of the present invention and the polysaccharide, the conjugate can be efficiently delivered to a Dictin-1 expressing antigen-presenting cell (e.g., macrophages, dendritic cells).

### BRIEF DESCRIPTION OF THE DRAWINGS

Fig. 1 shows the result obtained by confirming a purified dA40(S)-peptide conjugate by electrophoresis in Example 1.
Fig. 2 shows the result obtained by confirming the reactivity of aminopeptidase to various dA40(S)-peptide conjugates in Example 2.
Fig. 3 shows the result obtained by confirming the antigen presenting ability of various dA40(S)-peptide conjugates in Example 3.
Fig. 4 shows the result obtained by confirming the antigen presenting abilities of SPG/dA40(S)-OVA₂₅₇₋₂₆₄, dA40(S)-OVA₂₅₇₋₂₆₄, and OVA₂₅₇₋₂₆₄-click-dA40(S)/SPG in Example 5.
Fig. 5 shows the result obtained by performing an in vivo CTL assay using SPG/dA40(S)-OVA₂₅₇₋₂₆₄ in Example 6.

### EMBODIMENT OF THE INVENTION

The conjugate of the present invention is characterized in that the conjugate, where an N-terminal cysteine residue of an antigen peptide that has a cysteine residue at the N-terminal and has eight or more amino acid residues, is disulfide-bonded to a linker added to polydeoxyadenine. Hereinafter, the conjugate of the present invention will be described in detail.

### [Antigen Peptide]

The number of amino acid residues constituting the antigen peptide used in the present invention is 8 or more and is not limited as long as the antigen peptide is an antigen peptide having antigenicity (i.e., an antigen peptide which is recognized as a foreign substance by the body's immune system, thereby causing an immune response). The number is usually from 8 to 20, preferably from 8 to 12, and more preferably from 8 to 9.

The amino acid sequence of the antigen peptide used in the present invention is not particularly limited as long as the N-terminal of the amino acid sequence is a cysteine residue and the amino acid sequence can induce an immune response. Examples of the antigen peptide include antigen peptides having a partial amino acid sequence capable of acting as an epitope, among proteins as allergens (such as food allergies), and proteins derived from pathogens (such as bacteria and viruses) and tumor cells. Preferable examples of the antigen peptide include antigen peptides presented by MHC type I molecules. More specific examples of the antigen peptide used in the present invention include antigen peptides containing the amino acid sequences of the antigen peptides shown in Table 1. In the case of using the antigen peptides containing the amino acid sequences of the antigen peptides shown in Table 1, cysteine may be added to the N-terminal of each of the amino acid sequences of the antigen peptides shown in Table 1.

**[Table 1]**

| Antigen peptide | Sequence |
|---|---|
| OVA | SIINFEKL |
| Melanocyte gp100 for mouse | EGSRNQDWL |
| Melanocyte gp100 for human | KVPRNQDWL |
| TRP2 | SVYDFFVWL |
| CT26 | SPSYVYHQF |
| Influenza HA | JYSTVASSL |
| Influenza NP | ASNENMDTM |
| Influenza PA | SSLENFRAYV |
| β-galactosidase | DAPIYTNV |
| MuLV p15E | KSPWFTTL |
| SeV | FAPGNYPAL |
| MCMV IEI | YPHFMPTNL |
| LCMV gp33 | KAVYNFATM |
| LCMV NP396 | FQPQNGQFI |
| LCMV NP118 | RPQASGVYM |
| Malaria Pb9 | SYIPSAEKI |
| HIV P18-110 | RGPGRAFVTI |
| BCG MPT51 | GGPHAVYLL |
| Human CEA | EAQNTTYL |
| P815 | LPYLGWLVF |
| HBsAg | IPQSLDSWWTSL |
| HSV-1 gB | SSIEFARL |
| HY Uty | WMHHNMDLI |
| EGFP | HYLSTQSAL |
| HER2 | TYLPTNASL |
| VSV NP | RGYVYQGL |
| Polyomavirus MT | RRLGRTLLL |

In the amino acid sequences in the table, left end represents N-terminal and right end represents C-terminal

As long as the amino acid sequences of the antigen peptides shown in Table 1 are capable of inducing an immune response, the amino acid sequences may be those in which one or several amino acids are substituted, deleted, and/or inserted. In a case in which mutations such as substitution, deletion or insertion of amino acids are introduced into each of the amino acid sequences of the antigen peptides shown in Table 1, the number of the amino acid residues to which mutations are introduced may be appropriately set within a range where the immune response can be induced. The number is, for example, from 1 to 4, preferably from 1 to 3, more preferably 1 or 2, and particularly preferably 1.

Furthermore, in the amino acid sequences of the antigen peptides shown in Table 1, as long as the immune response can be induced, one or more amino acid residues may be added to at least one of the N-terminal and the C-terminal. In a case in which amino acid residues are added to the N-terminal and/or C-terminal of each of the amino acid sequences of the antigen peptides shown in Table 1, the number of the amino acid residues to be added may be appropriately set within a range where the immune response can be induced. The number is, for example, from 1 to 10, preferably from 1 to 5, and more preferably from 1 to 2. The antigen peptides preferably have a total length of 9 to 16 amino acid residues. Leucine, methionine, and phenylalanine are preferred as the amino acids to be added. Since the antigen peptide is recognized as an antigen from the C-terminal side, it is desirable that the amino acid residue is not added to the C-terminal side of each of the amino acid sequences of the antigen peptides shown in Table 1, except for the case where one hydrophobic amino acid residue to be described later is added.

As shown in Example 3 to be described later, it is confirmed that the conjugate of the present invention is taken up by the antigen-presenting cell, the C-terminal side of the antigen peptide is recognized, the C-terminal amino acid residue of the antigen peptide greatly affects the antigen presenting ability, and the antigen presentation is performed more efficiently when the C-terminal of the antigen peptide is a hydrophobic amino acid residue. Accordingly, the C-terminal of the amino acid sequence of the antigen peptide used in the present invention is preferably the hydrophobic amino acid residue. Specific examples of the hydrophobic amino acid residue include leucine, glycine, alanine, valine, isoleucine, methionine, proline, phenylalanine, and tryptophan. Among these hydrophobic amino acid residues, leucine is preferable.

In the case of using antigen peptides which contain the amino acid sequences of the antigen peptides shown in Table 1 and in which the C-terminal is the hydrophobic amino acid residue, regarding antigen peptides in which the C-terminal of each of the amino acid sequences of the antigen peptides shown in Table 1 is not the hydrophobic amino acid residue, one or more amino acid residues may be added so that the C-terminal is the hydrophobic amino acid residue, or one or more amino acid residues may be deleted from the C-terminal side so that the C-terminal is the hydrophobic amino acid residue.

The antigen peptide used in the present invention can be obtained by any known method such as hydrolysis of proteins as a source or peptide synthesis.

### [Polydeoxyadenine]

The number of deoxyadenines constituting the polydeoxyadenine used in the present invention may be appropriately set within a range where it is possible to form a complex with a polysaccharide having a β-1,3-glucan skeleton described later. The number is, for example, from 10 to 100, preferably from 20 to 100, more preferably from 20 to 80, and still more preferably from 30 to 50.

Further, at least a part of the phosphodiester bond of polydeoxyadenine used in the present invention may be phosphorothioated (S-modified). Polydeoxyadenine is S-modified in this manner, thereby imparting resistance against nuclease and making the stability in vivo more excellent. S-modification of polydeoxyadenine can be carried out in accordance with a conventionally known method.

In the case of S-modifying polydeoxyadenine used in the present invention, the S-modification ratio is not particularly limited, and is usually 50% or more, preferably 80% or more, and more preferably 100%. In the present specification, the S-modification ratio of polydeoxyadenine indicates a ratio (%) of S-modified phosphodiester bonds to the total number of phosphodiester bonds in polydeoxyadenine. Further, the term "S-modified phosphodiester bond" refers to a bond structure in which one of the oxygen atoms of the phosphate residue in the phosphodiester bond moiety is substituted with a sulfur atom.

### [Binding of Antigen Peptide to Polydeoxyadenine]

In the conjugate of the present invention, the thiol group of the N-terminal cysteine residue of the antigen peptide is disulfide-bonded to a linker (spacer) added to polydeoxyadenine.

In the conjugate of the present invention, either the 5' end side or the 3' end side of the polydeoxyadenine may be bonded to the N-terminal side of the antigen peptide via a linker, but the 5' end side of the polydeoxyadenine is preferably bonded to the N-terminal side of the antigen peptide via a linker.

In the conjugate of the present invention, the molecular weight of the linker moiety varies depending on the structure of the linker to be used, and the molecular weight is, for example, from 100 to 5000, preferably from 100 to 2000, and more preferably from 100 to 500.

The linker in the conjugate of the present invention may have a structure that is covalently bonded to the 5' end side or the 3' end side of the polydeoxyadenine and disulfide-bonded to the thiol group of the N-terminal cysteine residue of the antigen peptide. It is possible to use a linker obtained by linking one or two or more known bifunctional compounds.

In the conjugate of the present invention, the linkage between the polydeoxyadenine and the antigen peptide via a linker can be carried out, for example, by bonding a linker having a functional group capable of disulfide bonding to a thiol group to the 5' end side or the 3' end side of the polydeoxyadenine, and forming a disulfide bond between the thiol group of the N-terminal cysteine residue of the antigen peptide and the functional group.

In order to bond a linker having a functional group capable of forming a disulfide bond with a thiol group to the 5' end side or the 3' end side of the polydeoxyadenine, for example, a bifunctional compound having a functional group capable of covalently bonding to an amino group and a functional group capable of disulfide bonding to a thiol group may be bonded to polydeoxyadenine having an amino group added to the 5' end side or the 3' end side.

The polydeoxyadenine having an amino group added to the 5' end side or the 3' end side is, for example, polydeoxyadenine in which any of the groups of the following Formulae (1) to (7) is substituted with a hydrogen atom constituting the 5' end phosphate residue of polydeoxyadenine or a hydrogen atom constituting the hydroxyl group of the deoxyribonucleotide at the 3 'end of polydeoxyadenine.
[Formula 1]

-(CH₂)ₙ₁-NH₂ (1)

-(CH₂)ₙ₂-NH-CO-O-(CH₂)ₙ₃-NH₂ (2)

-CO-(CH₂)ₙ₁-NH₂ (3)

-NH-(CH₂)ₙ₁-NH₂ (4)

-CH(CH₂OH)ₙ₄-CH(CH₂CH₂O)-NH₂ (5)

-CH₂-(CH₂CH₂O)ₙ₄-NH₂ (6)

-CO-CH₂-(CH₂CH₂O)ₙ₄-NH₂ (7)

Here, in Formulae (1), (3), and (4), n1 is an integer of 1 to 18, preferably an integer of 1 to 12, and more preferably an integer of 1 to 6.

In Formula (2), n2 is an integer of 1 to 18, preferably an integer of 1 to 12, and more preferably an integer of 1 to 6; and n3 is an integer of 1 to 18, preferably an integer of 1 to 12, and more preferably an integer of 1 to 6.

In Formulae (5) to (7), n4 is an integer of 1 to 20, preferably an integer of 1 to 10, and more preferably an integer of 1 to 5.

The groups of Formulae (1) to (7) can be added to polydeoxyadenine in accordance with a known method.

Further, the bifunctional compound having a functional group capable of covalently bonding to an amino group and a functional group capable of disulfide bonding to a thiol group may be appropriately selected from known bifunctional crosslinking agents and used. Specific examples thereof include succinimidyl-6-[3'(2-pyridyldithio)propionato]hexanoate (LC-SPDP), N-succinimidyl-3-(2-pyridyldithio)propionate (SPDP), N-succinimidyl-S-acetylthioacetate (SATA), N-succinimidyl-S-acetylthiopropionate (SATP), and iminothiolane. Among these bifunctional linkers, succinimidyl-6-[3'(2-pyridyldithio)propionamido]hexanoate is preferable.

The bond between the polydeoxyadenine to which the amino group is added and the bifunctional compound as well as the disulfide bond between the bifunctional linker and the thiol group of the N-terminal cysteine residue can be formed by a known method.

### [Complexation with Polysaccharide Having β-1,3-glucan skeleton]

The conjugate of the present invention may be directly formulated as a vaccine. Desirably, the conjugate is used after forming a complex with the polysaccharide having a β-1,3-glucan skeleton. Thus, the conjugate is complexed with the polysaccharide having a P-1,3-glucan skeleton, whereby the conjugate can be efficiently delivered to the Dicten-1-expressing antigen-presenting cells (macrophages, dendritic cells, etc.). Accordingly, it is possible to obtain more excellent immunostimulatory effect.

β-1,3-glucan is a polysaccharide composed of glucoses linked via β1→3 glycosidic linkages, and there are various known polysaccharides in which the ratio of the number of glucose residues in the side chain to the number of glucose residues in the main chain (hereinafter abbreviated as "side chain ratio") varies. The polysaccharide having a β-1,3-glucan skeleton has helix parameters resembling those of nucleic acids such as poly(C) (e.g., see Takahashi, Obata, Suzuki, Prog. Polym. Phys. Jpn. 27, p. 767; and "Conformation of Carbohydrates", Sharwood Academic Publisher, 1998), and also has hydroxyl groups that are capable of hydrogen-bonding with nucleic acid bases. Thus, it is known that one polydeoxyadenine molecule and two polysaccharide molecules that have a P-1,3-glucan skeleton interact with one another to form a stable complex that has a triple helix structure.

Specific examples of the polysaccharide having a β-1,3-glucan skeleton used in the present invention include schizophyllan, curdlan, lentinan, pachyman, grifolan, and scleroglucan. Among them, schizophyllan is a preferable polysaccharide in the present invention, because it can significantly improve the introduction into the antigen-presenting cell and the resistance to enzymatic degradation. Further, in the case of using schizophyllan as the polysaccharide, the glucose residue of the side chain of schizophyllan is chemically modified, so that it is possible to introduce a functional group having an affinity for a cellular receptor (such as an amino group, an amino acid group, a peptide group or a cholesterol group).

The weight average molecular weight of the polysaccharide having a β-1,3-glucan skeleton is not particularly limited, and may be appropriately set according to the type of polysaccharide to be used and the chain length of polydeoxyadenine in the conjugate of the present invention. Specifically, the weight average molecular weight of the polysaccharide having a β-1,3-glucan skeleton is usually from 25,000 to 2,500,000, and preferably from 25,000 to 150,000.

The polysaccharide having a β-1,3-glucan skeleton can be obtained in accordance with a known method. For example, schizophyllan can be obtained in accordance with the standard method described in the literature (A. C. S. 38 (1), 253 (1997); Carbohydrate Research, 89, 121-135 (1981)).

Specifically, the conjugate of the present invention can be complexed with the polysaccharide having a β-1,3-glucan skeleton in the following manner. The polysaccharide having a P-1,3-glucan skeleton has a triple helix structure in nature or in water. The process of including: dissolving the polysaccharide in a polar solvent such as dimethyl sulfoxide (DMSO) or an alkaline aqueous solution such as an aqueous sodium hydroxide solution to cause disassociation of the triple helix into a single strand; adding the conjugate of the invention; and replacing the solvent with water or adjusting the pH of the alkaline aqueous solution to neutral (regenerating process) is performed, whereby two molecules of the polysaccharide having a β-1,3-glucan skeleton are bonded to the single-stranded moiety of the polydeoxyadenine linked to the conjugate of the present invention to form a triple helix complex structure (association structure). It is considered that the complex of the polydeoxyadenine and the polysaccharide is mainly formed through hydrogen bonding and hydrophobic interaction.

Desirably, complexation of the conjugate of the present invention and the polysaccharide having a β-1,3-glucan skeleton is carried out by mixing the conjugate with the polysaccharide at a molar ratio of the conjugate of the present invention to the polysaccharide having a β-1,3-glucan skeleton of 20 : 1 to 1 : 5, preferably 10 : 1 to 1 : 1. The molar ratio is used for the conditions of forming a complex of the conjugate of the present invention and the polysaccharide having a β-1,3-glucan skeleton, as a result of which both the conjugate and the polysaccharide efficiently interact to each other. This leads to improvement in the production efficiency of the complex of the conjugate of the present invention and the polysaccharide having a β-1,3-glucan skeleton.

### [Usage and Dosage Form]

The conjugate of the present invention and the complex of the conjugate of the present invention and the polysaccharide having a β-1,3-glucan skeleton can efficiently present antigen peptides in the antigen-presenting cells. Thus, the conjugate and the complex can be formulated as vaccine formulations for treating and preventing infectious diseases caused by infection of pathogens (e.g., bacteria and viruses) and for tumors such as cancer.

The vaccine formulation can be prepared by mixing the conjugate of the present invention or the complex of the conjugate of the present invention and the polysaccharide having a β-1,3-glucan skeleton, as appropriate, using a pharmaceutically acceptable carrier or excipient, and formulating the mixture in accordance with a known formulation technique. The dosage form of the vaccine formulation is not particularly limited, and examples thereof include tablets, suppositories, capsules, syrups, microcapsules, injectable solutions, aerosols, and sprays.

The vaccine formulation can be orally or parenterally administered to humans or warm-blooded animals (such as mice, rats, rabbits, sheep, pigs, cattle, horses, chickens, cats, dogs, and monkeys). Examples of parenteral administration include subcutaneous injection, intradermal injection, intramuscular injection, intraperitoneal administration, intravenous drip, nasal mucous membrane spray administration, and pharyngeal region spray administration.

The dose of the vaccine formulation may be appropriately set according to activity, the disease to be treated, the type, body weight, sex, and age of the animal to be administered, severity of the disease, administration method, and the like. For example, in the case of administration to an adult, the daily dose may be from about 1 to about 50,000 µg, and preferably from about 10 to about 10000 µg in terms of weight of antigen peptide.

If necessary, the vaccine formulation may contain a CpG oligodeoxynucleotide adjuvant, a Freund's adjuvant, an aluminum hydroxide (Alum) adjuvant, and an alum adjuvant, and the like. Alternatively, the vaccine formulation may be administered together with these adjuvants.

### EXAMPLES

Hereinafter, the present invention will be described in more detail with reference to examples, but the present invention is not limited to these examples. In this regard, 40-mer polydeoxyadenine having an S-modification ratio of 100% may be hereinafter referred to as "dA40(S)".

### Example 1: Preparation of conjugate of peptide and dA40(S)

1 mol of dA40(S) having an amino group added to the 5' end (40-mer polydeoxyadenine in which a hydrogen atom constituting the phosphate residue at the 5' end was substituted with the -(CH₂)₆-NH-CO-O-(CH₂)₂-NH₂ group and which had an S-modification ratio of 100%) and 30 mol equivalent of succinimidyl-6-[3'(2-pyridyldithio)propionamido]hexanoate (LC-SPDP) were mixed in a phosphate buffer solution (PBS), and the mixture was reacted at 40°C for 3 hours.

Then, unreacted LC-SPDP was removed using an anion exchange column (NAP-5 column) to obtain dA40(S)-SPDP in which LC-SPDP was bonded to the 5' end of dA40(S).

25 mol equivalent of peptides 1 to 4 (peptides 1 to 3, i.e., ovalbumin (OVA) antigen peptides) shown in Table 2 was added to 1 mol of dA40(S)-LC-SPDP, and the mixture was reacted at 40°C for 18 hours.

**[Table 2]**

| | Amino acid sequence |
|---|---|
| Peptide 1 | SIINFEKLC (SEQ ID NO: 1) |
| Peptide 2 | CSIINFEKL (SEQ ID NO: 2) |
| Peptide 3 | CQLESIINFEKL (SEQ ID NO: 3) |
| Peptide 4 | CSIINFEKK (SEQ ID NO: 4) |

Thereafter, the reaction solution was subjected to HPLC to separate the conjugate of dA40(S) and peptide. The HPLC measurement conditions were set as follows:
Solution A: acetonitrile
Solution B: 0.1 M TEAA (pH 7.0)
Column: ZORBAX Eclipse Plus C18
Eluent
0 min Solution A: 10%,Solution B: 90%

| | | |
|---|---|---|
| to 25 min | 30%, | 70% |
| to 30 min | 100%, | 0% |
| to 32 min | 100%, | 0% |
| to 35 min | 10%, | 90% |

The separated solution was exchanged with distilled water using an Amicon Ultra Ultracel-3K filter and the resulting solution was freeze-dried to obtain a dA40(S)-peptide conjugate. Purification of the resulting dA40(S)-peptide conjugate was confirmed by acrylamide gel electrophoresis.

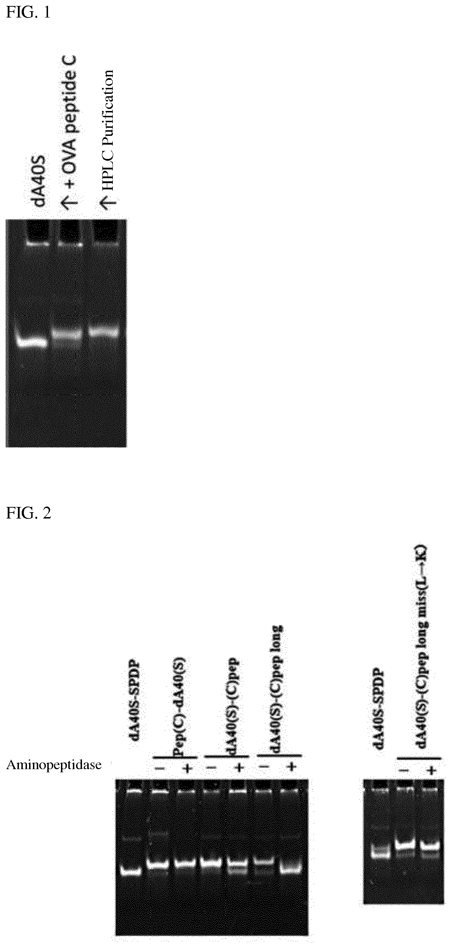

In the case of using peptide 2, the conjugate was subjected to electrophoresis, the stained conjugate was stained with SYBR Gold, and a fluorescent image thereof was obtained. The result is shown in Fig. 1. In Fig. 1, the left lane shows the result of dA40(S) alone, the middle lane shows the result of the product after reacting the peptide with dA40(S), and the right lane shows the result of the purified dA40(S)-peptide conjugate. From Fig. 1, it is understood that, in the lane (middle) showing the result of the product after reacting the peptide with dA40(S), the band of dA40(S) moves to the polymer side, and the peptide is bound to dA40(S). However, unreacted dA40(S) was observed, but only the band of the conjugate was observed after HPLC separation. This result confirmed that the dA40(S)-peptide conjugate was synthesized and purified.

### Example 2: Cleavage of dA40(S)-peptide conjugate by endoplasmic reticulum aminopeptidase

Each of the dA40(S)-peptide conjugates (1.5 µM) and murine endoplasmic reticulum aminopeptidase (0.5 µg/ml) (R & D Systems catalog Number 2500-ZN) were added to a 25 mM Tris buffer (pH 8.0) and the mixture was incubated at 37°C for 18 hours. After completion of the reaction, polyacrylamide gel electrophoresis was carried out to observe the state of release from the dA40(S)-peptide conjugate.

Fig. 2 shows the results of polyacrylamide gel electrophoresis. In Fig. 2, pep(C)-dA40(S) represents a dA40(S)-peptide conjugate obtained by using peptide 1, dA40(S)-(C)pep represents a dA40(S)-peptide conjugate obtained by using peptide 2, dA40(S)-(C)pep long represents a dA40(S)-peptide conjugate obtained by using peptide 3, and dA40(S)-(C)pep long miss (L→K) represents a dA40(S)-peptide conjugate obtained by using peptide 4. In the conjugate in which dA40(S) was bonded, via a disulfide bond, to the C-terminal cysteine residue of peptide 1 having a cysteine residue at the C-terminal, no change was observed in the electrophoretic distance even when subjected to enzyme treatment. In other words, it was found that the dA40(S)-peptide conjugate in this orientation was hardly recognized by the enzyme. Conversely, in the conjugate in which dA40(S) was bonded, via a disulfide bond, to the N-terminal cysteine residue of peptide 2 having a cysteine residue at the N-terminal, a band of dA40(S) was slightly observed after the enzyme treatment. Further, in peptide 3 in which the number of amino acids was increased as compared to peptide 2, the peptide was almost completely released and a band of dA40(S) was observed. These facts shows that dA40(S) is bonded, via a disulfide bond, to the N-terminal cysteine residue of the antigen peptide, whereby the antigen peptide and dA40(S) can be cleaved by aminopeptidase. Further, the dA40(S)-peptide conjugate obtained by using peptide 4 in which the C-terminal of peptide 2 was substituted with leucine from lysine was not recognized by the enzyme. Aminopeptidase is one of the enzymes that trim antigen peptides to present the antigen peptides to MHC. Accordingly, it is important for antigen presentation using the dA40(S)-peptide conjugate that even in the case of the peptide in which polydeoxyadenine is bonded to the N-terminal cysteine, the C-terminal side is composed of the hydrophobic amino acid residue. This fact was confirmed.

### Example 3: Evaluation of antigen presenting ability using dA40(S)-peptide conjugate

3 ml of thioglycollate medium was administered to the peritoneal cavity of C57BL/6J mice 3 days later, macrophages were recovered from the peritoneal cavity. The resulting macrophages were seeded in 48 wells (1.5 × 10⁵ cells/well), and further each of the dA40(S)-peptide conjugates was added to the wells at a concentration of 10 µM. Cells were fixed with 4% paraformaldehyde in a CO₂ incubator for 18 hours, the fixed cells were stained with Anti-Mouse OVA257-264 (SIINFEKL) peptide bound to H-2Kb PE, and the resulting cells were subjected to fluorescence microscopic observation.

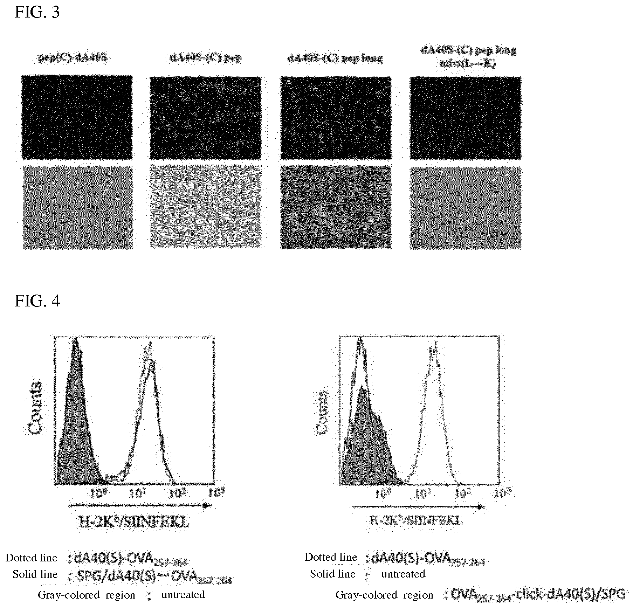

Fig. 3 shows the results of fluorescence microscopic observation. In Fig. 3, pep(C)-dA40(S) represents a dA40(S)-peptide conjugate obtained by using peptide 1, dA40(S)-(C)pep represents a dA40(S)-peptide conjugate obtained by using peptide 2, dA40(S)-(C)pep long represents a dA40(S)-peptide conjugate obtained by using peptide 3, and dA40(S)-(C)pep long miss (L→K) represents a dA40(S)-peptide conjugate obtained by using peptide 4. Very weak fluorescence was observed in the dA40(S)-peptide conjugate obtained by using peptide 1. Further, very strong fluorescence was observed at about the same degree in the dA40(S)-peptide conjugate obtained by using peptides 2 and 3. Further, antigen presentation hardly occurred in the dA40(S)-peptide conjugate obtained by using peptide 4. These results indicate that the dA40(S)-antigen peptide conjugate is introduced into the antigen-presenting cells, the C-terminal side of the dA40(S)-antigen peptide conjugate is recognized, and it is important for the antigen presentation that the C-terminal is the hydrophobic amino acid residue.

### Example 4: Preparation of complex of dA40(S)-peptide conjugate and schizophyllan

Ovalbumin (OVA) antigen peptide (OVA₂₅₇₋₂₆₄; amino acid sequence: SIINFEKL) was used to prepare a dA40(S)-OVA₂₅₇₋₂₆₄ conjugate (dA40(S)-OVA₂₅₇₋₂₆₄) in the manner described in Example 1. Schizophyllan (SPG) was dissolved in a 0.25 N aqueous sodium hydroxide solution for 2 to 5 days to obtain an SPG solution in which the triple helix structure was dissociated. Next, the SPG solution in which the triple helix structure was dissociated, the dA40(S)-OVA₂₅₇₋₂₆₄ conjugate, and a phosphate buffer solution (330 mM NaH₂PO₄, pH 4.7) were mixed, and the mixture was allowed to stand at 4°C overnight. In the mixture, the concentration of dA40(S)-OVA₂₅₇₋₂₆₄ was 60 µM, and the ratio of [dA40(S)-OVA₂₅₇₋₂₆₄]/[SPG] was 4. In this manner, a complex (SPG/dA40(S)-OVA₂₅₇₋₂₆₄) of dA40(S)-OVA₂₅₇₋₂₆₄ and SPG was prepared.

### Reference Example 1: Preparation of complex of CpG-dA40(S) and SPG

CpG-dA40(S) in which dA40(S) was ligated to the 3' end of CpG (base sequence: ATCGACTCTCGAGCGTTCTC) was provided. CpG-dA40(S) was used to form a complex (CpG-dA40(S)/SPG) of CpG-dA40(S) and SPG, in the same manner as in Example 4.

### Reference Example 2: Preparation of conjugate of dA40(S) and OVA₂₅₇₋₂₆₄ shown in Patent Document 1 (International Publication No. WO 2015/084374) and preparation of complex of the conjugate and SPG

Ovalbumin (OVA) antigen peptide (OVA₂₅₇₋₂₆₄; amino acid sequence: SIINFEKL) (OVA (N3) described below) having an azido group introduced into the C-terminal was reacted with dA40(S) (dA40(S) described below) (alkyne) having alkyne introduced into the 5' end according to the method described in Example 1 of Patent Document 1 (International Publication No. WO 2015/084374) to cause a cycloaddition reaction between the azide group and the alkyne, thereby preparing a conjugate (OVA₂₅₇₋₂₆₄-click-dA40(S)) of dA40(S) and OVA₂₅₇₋₂₆₄.

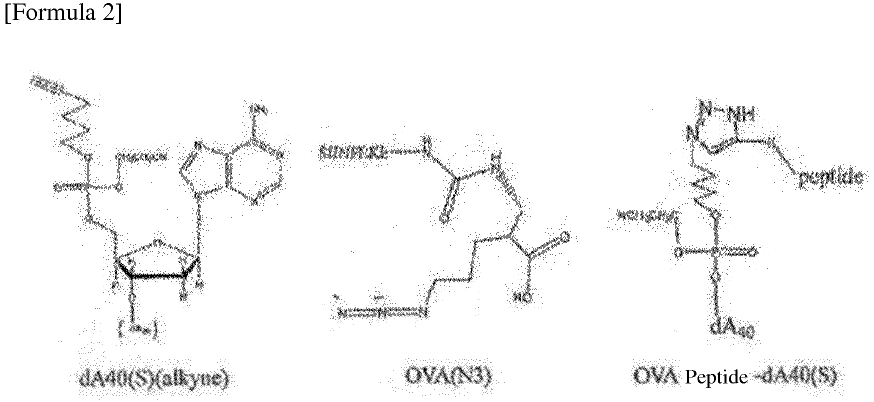

Subsequently, OVA₂₅₇₋₂₆₄-click-dA40(S) was used to form a complex (OVA₂₅₇₋₂₆₄-click-dA40(S)/SPG) of OVA₂₅₇₋₂₆₄-click-dA40(S) and SPG, in the same manner as in Example 4.

### Example 5: Evaluation of antigen presenting ability using SPG/dA40(S)-OVA₂₅₇₋₂₆₄

3 ml of thioglycollate medium was administered to the peritoneal cavity of C57BL/6J mice 3 days later, macrophages were recovered from the peritoneal cavity. The resulting macrophages were seeded in 48 wells (1.5 × 10⁵ cells/well), and further SPG/dA40(S)-OVA₂₅₇₋₂₆₄, dA40(S)-OVA₂₅₇₋₂₆₄, or OVA₂₅₇₋₂₆₄-click-dA40(S)/SPG was added at a concentration of OVA₂₅₇₋₂₆₄ of 5 µg/ml. Cells were fixed with 4% paraformaldehyde in a CO₂ incubator for 18 hours, the fixed cells were stained with Anti-Mouse OVA257-264 (SIINFEKL) peptide bound to H-2Kb PE, and the resulting cells were analyzed by flow cytometry.

The results are shown in Fig. 4. The antigen (OVA₂₅₇₋₂₆₄) presentation level of dA40(S)-OVA₂₅₇₋₂₆₄ was the same as that of SPG/dA40(S)-OVA₂₅₇₋₂₆₄. On the other hand, antigen presentation did not occur in OVA₂₅₇₋₂₆₄-click-dA40(S)/SPG. This suggests that the peptide was released from the complex in the cell (i.e., the disulfide bond was cleaved in a reducing environment), whereby the subsequent antigen presentation was efficiently carried out.

### Example 6: In vivo CTL assay

SPG/dA40(S)-OVA₂₅₇₋₂₆₄ (20, 100, 500 ng in the amount of OVA₂₅₇₋₂₆₄) and CpG-dA40(S)/SPG (30 µg in the amount of CpG-dA) were intradermally administered to C57BL/6J mice.

On day 7 of administration, spleen cells were provided according to the following procedure. Spleen cells excised from normal C57BL/6J mice were treated with 10 µg/ml of OVA₂₅₇₋₂₆₄ at 37°C for 90 minutes. Thereafter, the spleen cells were washed three times with PBS, and the spleen cells were labeled with 5 µM carboxyfluorescein succinimidyl ester (CFSE); Invitrogen, Carlsbad, Calif.). Further, spleen cells not treated with OVA₂₅₇₋₂₆₄ were similarly labeled with CFSE (0.5 µM). The labeled cells were washed three times with PBS. The spleen cells treated with OVA₂₅₇₋₂₆₄ (CFSE^{high}) and the spleen cells not treated with OVA₂₅₇₋₂₆₄ (CFSE^{low}) were mixed so that the number of the treated spleen cells was equal to the number of the untreated spleen cells. The resulting spleen cells were administered intravenously (4.0 × 10⁶ cells/head) into mice on Day 7 of administration of SPG/dA40(S)-OVA₂₅₇₋₂₆₄ and CpG-dA40(S)/SPG, and the mice were bred for 24 hours. After that, the spleen cells were removed from the mice, and the CFSE-labeled cells were counted by flow cytometry.

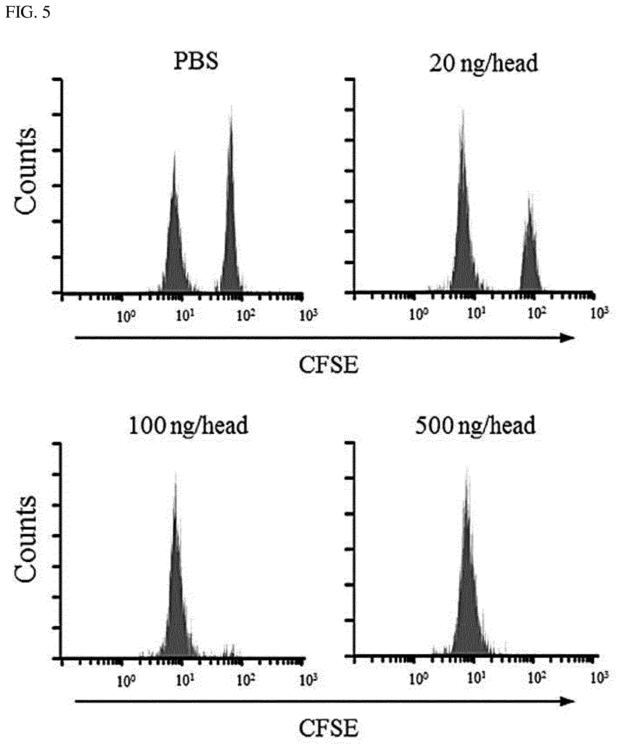

The results are shown in Fig. 5. In mice immunized with SPG/dA40(S)-OVA₂₅₇₋₂₆₄ and CpG-dA40(S)/SPG, the decrease in OVA₂₅₇₋₂₆₄ presenting cells (CFSE^{high} spleen cells) was observed depending on the dose of OVA₂₅₇₋₂₆₄. OVA₂₅₇₋₂₆₄ presenting cells were not observed when the dose of OVA₂₅₇₋₂₆₄ was 100 ng/head or more. This showed that immunized mice specifically eliminated only the antigen-containing cells. It is reported that, in OVA₂₅₇₋₂₆₄-click-dA40(S)/SPG, administration of at least 2 µg/head of OVA₂₅₇₋₂₆₄ is required to induce the CTL response (Mochizuki, S., Morishita, H., Kobiyama, K., Aoshi, T., Ishii, K. J., and Sakurai, K. (2015) Immunization with antigenic peptides complexed with beta-glucan induces potent cytotoxic T-lymphocyte activity in combination with CpG-ODNs. J. Control. Release 220, 495-502). Hence, it is revealed that SPG/dA40(S)-OVA₂₅₇₋₂₆₄ can induce high CTL response with a smaller peptide dose compared to OVA₂₅₇₋₂₆₄-click-dA40(S)/SPG. The difference in ability of inducing CTL reaction between SPG/dA40(S)-OVA₂₅₇₋₂₆₄ and OVA₂₅₇₋₂₆₄-click-dA40(S)/SPG was consistent with the result of Example 5 in which the antigen presenting ability was evaluated.

## Claims

1. A nucleic acid-peptide conjugate, where an N-terminal cysteine residue of an antigen peptide that has a cysteine residue at the N-terminal and has eight or more amino acid residues, is disulfide-bonded to a linker added to polydeoxyadenine.

2. The nucleic acid-peptide conjugate according to claim 1, wherein a C-terminal amino acid residue of the antigen peptide is a hydrophobic amino acid residue.

3. The nucleic acid-peptide conjugate according to claim 1 or 2, wherein the antigen peptide is a peptide presented by an MHC class I molecule.

4. The nucleic acid-peptide conjugate according to any one of claims 1 to 3, wherein at least a part of a phosphodiester bond of the polydeoxyadenine is phosphorothioated (S-modified).

5. A complex comprising:
the nucleic acid-peptide conjugate according to any one of claims 1 to 4; and
a polysaccharide having a β-1,3-glucan skeleton.

6. A vaccine formulation comprising:
the nucleic acid-peptide conjugate according to any one of claims 1 to 4; or
the complex according to claim 5.

7. An immunization method comprising administering the nucleic acid-peptide conjugate according to any one of claims 1 to 4 or the complex according to claim 5, to a subject who needs to be given immunity or needs to enhance immunity.

8. Use of the nucleic acid-peptide conjugate according to any one of claims 1 to 4, or the complex according to claim 5, for the vaccine formulation.


SEQUENCE LISTING

<110>  NapaJen Pharma, Inc.

<120>  Conjugate of nucleic acid and antigen peptide

<130>  17067WO

<150>  JP2016-120261
<151>  2016-06-16

<160>  4

<170>  PatentIn version 3.5

<210>  1
<211>  9
<212>  PRT
<213>  Artificial Sequence

<220>
<223>  antigen peptide 1

<400>  1

Ser Ile Ile Asn Phe Glu Lys Leu Cys
1               5


<210>  2
<211>  9
<212>  PRT
<213>  Artificial Sequence

<220>
<223>  antigen Peptide 2

<400>  2

Cys Ser Ile Ile Asn Phe Glu Lys Leu
1               5


<210>  3
<211>  12
<212>  PRT
<213>  Artificial Sequence

<220>
<223>  antigen peptide 3

<400>  3

Cys Gln Leu Glu Ser Ile Ile Asn Phe Glu Lys Leu
1               5                   10


<210>  4
<211>  9
<212>  PRT
<213>  Artificial Sequence

<220>

<223>  antigen peptide 4

<400>  4

Cys Ser Ile Ile Asn Phe Glu Lys Lys
1               5